(19) Europäisches Patentamt
European Patent Office
Office européen des brevets

(11) EP 1 810 696 A1

(12) **EUROPEAN PATENT APPLICATION**
published in accordance with Art. 158(3) EPC

(43) Date of publication:
**25.07.2007 Bulletin 2007/30**

(21) Application number: **05800033.2**

(22) Date of filing: **27.10.2005**

(51) Int Cl.:
***A61K 48/00*** (2006.01) ***A61K 38/00*** (2006.01)
***A61P 9/00*** (2006.01) ***A61P 9/04*** (2006.01)
***A61P 9/10*** (2006.01)

(86) International application number:
**PCT/JP2005/020163**

(87) International publication number:
**WO 2006/046766 (04.05.2006 Gazette 2006/18)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.10.2004 JP 2004317329**

(71) Applicants:
- **AnGes MG, Inc.**
  **Ibaraki-shi,**
  **Osaka 567-0085 (JP)**
- **Sawa, Yoshiki**
  **Hyogo, 6620099 (JP)**

(72) Inventors:
- **SHIRAKAWA, Yukitoshi**
  **c/o ANGES MG, INC.**
  **Ibaraki-shi, Osaka 5670085 (JP)**
- **TATSUMI, Eisuke**
  **c/o ANGES MG, INC.**
  **Ibaraki-shi, Osaka 5670085 (JP)**
- **TAENAKA, Yoshiyuki**
  **c/o ANGES MG, INC.**
  **Ibaraki-shi, Osaka 5670085 (JP)**
- **MATSUDA, Hikaru**
  **c/o ANGES MG, INC.**
  **Ibaraki-shi, Osaka 5670085 (JP)**

(74) Representative: **HOFFMANN EITLE**
**Patent- und Rechtsanwälte**
**Arabellastrasse 4**
**81925 München (DE)**

# (54) GENE THERAPY FOR TREATMENT OF CARDIAC FAILURE

(57) The present invention provides a medicament comprising a gene encoding an angiogenic cytokine for the treatment of acute myocardial infarction (AMI), idiopathic cardiomyopathy (ICM), dilated cardiomyopathy (DCM) or heart failure, to be given in combination with ventricular assist device (VAD).

EP 1 810 696 A1

# SEQUENCE LISTING

<110> Anges MG, Inc

<120> Gene therapy for the treatment of heart failure

<130> 05100PCT

<160> 1

<210> 1
<211> 2187
<212> DNA
<213>Homo sapiens

<220>
<223> hHGFcDNA

<400> 1

```
atgtgggtga ccaaactcct gccagccctg ctgctgcagc atgtcctcct gcatctcctc      60
ctgctcccca tcgccatccc ctatgcagag ggacaaagga aaagaagaaa tacaattcat     120
gaattcaaaa aatcagcaaa gactacccta atcaaaatag atccagcact gaagataaaa     180
accaaaaaag tgaatactgc agaccaatgt gctaatagat gtactaggaa taaaggactt     240
ccattcactt gcaaggcttt tgtttttgat aaagcaagaa aacaatgcct ctggttcccc     300
ttcaatagca tgtcaagtgg agtgaaaaaa gaatttggcc atgaatttga cctctatgaa     360
aacaaagact acattagaaa ctgcatcatt ggtaaaggac gcagctacaa gggaacagta     420
tctatcacta agagtggcat caaatgtcag ccctggagtt ccatgatacc acacgaacac     480
agctttttgc cttcgagcta tcggggtaaa gacctacagg aaaactactg tcgaaatcct     540
cgaggggaag aagggggacc ctggtgtttc acaagcaatc cagaggtacg ctacgaagtc     600
tgtgacattc ctcagtgttc agaagttgaa tgcatgacct gcaatgggga gagttatcga     660
ggtctcatgg atcatacaga atcaggcaag atttgtcagc gctgggatca tcagacacca     720
caccggcaca aattcttgcc tgaaagatat cccgacaagg gctttgatga taattattgc     780
cgcaatcccg atggccagcc gaggccatgg tgctatactc ttgaccctca cacccgctgg     840
gagtactgtg caattaaaac atgcgctgac aatactatga atgacactga tgttcctttg     900
gaaacaactg aatgcatcca aggtcaagga gaaggctaca ggggcactgt caataccatt     960
tggaatggaa ttccatgtca gcgttgggat tctcagtatc ctcacgagca tgacatgact    1020
cctgaaaatt tcaagtgcaa ggacctacga gaaaattact gccgaaatcc agatgggtct    1080
gaatcaccct ggtgttttac cactgatcca aacatccgag ttggctactg ctcccaaatt    1140
ccaaactgtg atatgtcaca tggacaagat tgttatcgtg ggaatggcaa aaattatatg    1200
ggcaacttat cccaaacaag atctggacta acatgttcaa tgtgggacaa gaacatggaa    1260
gacttacatc gtcatatctt ctgggaacca gatgcaagta agctgaatga gaattactgc    1320
cgaaatccag atgatgatgc tcatggaccc tggtgctaca cgggaaatcc actcattcct    1380
tgggattatt gccctatttc tcgttgtgaa ggtgatacca cacctacaat agtcaattta    1440
gaccatcccg taatatcttg tgccaaaacg aaacaattgc gagttgtaaa tgggattcca    1500
acacgaacaa acataggatg gatggttagt ttgagataca gaaataaaca tatctgcgga    1560
ggatcattga taaaggagag ttgggttctt actgcacgac agtgtttccc ttctcgagac    1620
ttgaaagatt atgaagcttg gcttggaatt catgatgtcc acggaagagg agatgagaaa    1680
tgcaaacagg ttctcaatgt ttcccagctg gtatatggcc ctgaaggatc agatctggtt    1740
ttaatgaagc ttgccaggcc tgctgtcctg gatgattttg ttagtacgat tgatttacct    1800
aattatggat gcacaattcc tgaaaagacc agttgcagtg tttatggctg gggctacact    1860
ggattgatca actatgatgg cctattacga gtggcacatc tctatataat gggaaatgag    1920
aaatgcagcc agcatcatcg agggaaggtg actctgaatg agtctgaaat atgtgctggg    1980
gctgaaaaga ttggatcagg accatgtgag ggggattatg gtggcccact tgtttgtgag    2040
caacataaaa tgagaatggt tcttggtgtc attgttcctg gtcgtggatg tgccattcca    2100
aatcgtcctg gtatttttgt ccgagtagca tattatgcaa aatggataca caaaattatt    2160
ttaacatata aggtaccaca gtcatag                                        2187
```

## Description

Technical Field

The present invention relates to gene therapy for the treatment of heart failure or to a medicament thereof.

Background Art

Reference numbers parenthesized, inserted in the description, indicate references identified at the end of the description.

Cardiac transplantation continues to be the destination therapy for patients with severe congestive heart failure (CHF).

However, the overall applicability of cardiac transplantation is limited by a severe shortage of donors (1).

For many patients with severe CHF, pharmacological therapy is insufficient, and revascularization or other surgical procedures are usually only palliative and do not greatly reduce the overall ultimate mortality.

Left ventricular assist devices (LVAD) are being used with greater frequency to provide circulatory support until transplantation can be achieved.

Unfortunately, many patients are now spending several months and even years on these devices.

Although improvements in LVAD have resulted in clinically meaningful survival benefits and an improved quality of life for patients with severe CHF, further improvements are needed (2).

There have been several recent reports of selected patients with end-stage CHF whose recovery of cardiac function by LVAD was sufficient that the device could be explanted successfully (3-5).

However, such patients constitute only a small percentage of patients using LVAD.

And the long-term outcome of recovery, the mechanism of recovery, and which patients are capable of recovery remain unclear.

Because the number of patients with severe CHF continues to increase, there have been several efforts to seek alternatives, such as regeneration therapy.

Hepatocyte Growth Factor (HGF) is a potent angiogenic agent possessing mitogenic, motogenic, and morphogenic effects through its own specific receptor, c-Met, in various types of cells, including myocytes (6, 7).

We have previously demonstrated that HGF exerts antifibrotic and antiapoptotic effects in the myocardium (8-10).

Considering the pathogenic characteristics of severe heart failure, such as progression of fibrosis, progression of endothelial dysfunction, loss of functional capillaries, and apoptosis-related loss of contractile mass (11, 12), HGF might have a beneficial effect in the impaired heart by attenuating these remodeling processes (13, 14).

Therefore, gene transfection with the HGF gene may enable a "bridge to recovery" in the impaired heart under the support of LVAD.

To investigate this possibility, we performed gene therapy with HGF in impaired goat hearts implanted with LVAD.

WO01/026694 disclosed a method for repairing cardiac function by noninvasive administration of an HGF gene in the form of Sendai virus (HVJ)-liposome into the affected cardiac muscle.

However, this prior art did not disclose neither naked plasmid administration nor combination use with ventricular assist device (VAD).

Disclosure of the Invention

The present invention relates to each of the followings:

(1) a medicament comprising a gene encoding an angiogenic cytokine for the treatment of acute myocardial infarction (AMI), idiopathic cardiomyopathy (ICM), dilated cardiomyopathy (DCM) or heart failure, to be given in combination with ventricular assist device (VAD);

(9) a method of treating acute myocardial infarction (AMI), idiopathic cardiomyopathy (ICM), dilated cardiomyopathy (DCM) or heart failure, which comprises administering the above-mentioned medicament to a patient using ventricular assist device (VAD) in combination; or

(10) use of the gene encoding an angiogenic cytokine for producing a medicament for treating acute myocardial infarction (AMI), idiopathic cardiomyopathy (ICM), dilated cardiomyopathy (DCM) or heart failure of a patient using ventricular assist device (VAD) in combination.

Brief Description of Drawings

Fig. 1 is a photograph showing animal model of the impaired heart and experimental design.

A? Creation of myocardial infarction in adult goat hearts by ligating the left anterior descending coronary artery (LAD) and provides direct administration (crosses) of plasmid encoding hHGF cDNA or beta-galactosidase into the myocardium.
B, Bi-ventricular assist devices (BVAD) installed in all goats of the impaired heart.
C, Photograph showing the site of ultrasonic crystals.
Two ultrasonic crystals were implanted in the endocardium parallel to the short axis of the left ventricle at the level of papillary muscle as figures at the time of operation.

Fig. 2 is graphs showing comparison of VAD assist ratio (A), and a group showing comparison of native cardiac output (B) of the HGF group (squares) and the control group (circles) through this experiment.
Data are presented as mean±SD.
*P<0.01 vs. the control group.
Fig. 3 is diagrams showing comparison of wall contractile function evaluated by percent fractional shortening (%FS) which was calculated by the sonomicrometry method.
The HGF group was squares and the control group was circles. Data are presented mean±SD.
*P<0.01 versus the control group.
Fig. 4 is a diagram showing changes of hemodynamic conditions after weaning from VAD.
Heart rate (A, HR), systemic blood pressure (B, mean AoP), mixed venous oxygen saturation (C, Sv02) and pulmonary arterial pressure (D, mean PAP), native cardiac output (E, CO) and left ventricular end-diastolic dimension (F, LVDd) was measured. Data are presented mean±SD.
*P<0.05 versus the control group.
Fig. 5 is a photograph showing histological findings of the heart 4 weeks after gene transfection.
A, B, Macroscopic findings of short axis area of the left ventricle.
C, D, Azan-trichrome staining of the myocardium in the border zone of the infracted and normal area (bar=100 μm, original magnification ×100).
E, F, Hematoxylin and eosin staining of the myocardium of the border zone (bar=100 μm, original magnification ×200,).
G, H, Immunohistologic staining by von Willebrand antibody (bar=100 μm, original magnification ×200).
Arrows indicated the example of von Willebrand antibody positive endothelial cells.
A, C, E and G were the HGF group.
B, D, F and H were the control group.
Fig. 6 is a graph showing evaluation of histopathological findings.
A, Percent fibrosis
B, cell diameter of myocyte in the border zone.
Data are represented mean±SD.
C, Vascualr density.
Arteriole density

Detailed Description of the invention

Specifically, the present invention also includes the followings:

(2) the medicament described in (1), wherein the angiogenic cytokine is selected from the group consisting of hepatocyte growth factor (HGF), vascular endothelial growth factor (VEGF), fibroblast growth factor (FGF), epidermal growth factor (EGF), nerve growth factor (NGF), transforming growth factor (TGF), platelet derived growth factor (PDGF) and insulin-like growth factor (IGF);
(3) the medicament described in (1) or (2), wherein the angiogenic cytokine is HGF;
(4) the medicament described in any of (1) to (3), wherein the gene is in the form of plasmid;
(5) the medicament described in any of (1) to (4), wherein the angiogenic cytokine is administered directly into the myocardium;
(6) the medicament described in any of (1) to (5), wherein the angiogenic cytokine is injected at plural points in the myocardium;

(7) the medicament described in any of (1) to (6), wherein the myocardium is the ischemic area of the left ventricular wall; and

(8) the medicament described in any of (1) to (7), wherein the VAD is left ventricular assist device (LVAD).

Although Left Ventricular Assist Device (LVAD) is often used to provide circulatory support until transplantation in severe heart failure, the mortality of long-term LVAD remains high. We have revealed that Hepatocyte Growth Factor (HGF) has effects of angiogenesis, antifibrosis and antiapoptosis in the myocardium. Therefore, gene therapy using HGF-cDNA plasmid may enhance the chance of "bridge to recovery". In this study, we performed gene therapy with HGF in the impaired goat heart under LVAD. Specifically, six adult goats (56-65kg) were created the impaired heart by ligating the coronary artery and installed VAD. The HGF group (n=3) administered human HGF-cDNA plasmid of 2.0 mg in myocardium. The control group (n=3) administered beta-galactosidase plasmid similarly. Four weeks after gene transfection, all goats tried to wean from VAD.

The myocardia transfected with the hHGF-cDNA contained hHGF protein at levels as high as 1.0$\pm$0.3 ng / g tissue 3 days after transfection. After weaning from VAD, the HGF group showed good hemodynamics while the control group showed its deterioration. The percent fractional shortening was significantly higher in the HGF group than the control group (HGF vs. control, 37.9$\pm$1.7 % vs. 26.4$\pm$0.3 %, $p < 0.01$). LV dilatation associated with myocytes hypertorophy and fibrotic changes were detected in the control group while not in the HGF group. Vascular density was markedly increased in the HGF group. These results suggest that gene therapy using hHGF may enhance the chance of "bridge to recovery" in the impaired heart under VAD.

## Examples

The present invention is further illustrated by the following examples, but not limited thereto.

### (1) Methods

#### 1) Preparation of plasmid encoded human HGF cDNA

A human HGF (hHGF) sequence shown in SEQ ID NO: 1 was inserted into the Not I site of the pUC-SR$\alpha$ expression vector plasmid as described elsewhere [15].

In this plasmid, expression of the hHGF cDNA is regulated under the control of the SR$\alpha$ promoter which is composed of simian virus 40 polyadenylation sequence.

The purified plasmid containing 2000 $\mu$g of hHGF-cDNA was reconstituted in sterile saline 2.0 ml and was directly injected into the myocardium at ten points with a 2.5 ml-syringe and 30 gauge-needle.

The concentration of hHGF in the heart was determined by enzyme-linked immunosorbent assay (ELISA) with anti-human HGF antibody (Institute of Immunology, Tokyo, Japan).

The antibody against hHGF reacts with only hHGF and not with goat HGF.

The serum hHGF levels were also assessed with the same ELISA system at 1, 3, 5, 7, 14, 28 days after cDNA injection.

### (2) Animal model of heart impairment

Ten adult goats weighing 56 to 65 kg were used for this study.

All animals were treated humanely in compliance with the "Principles of Laboratory Animal Care" formulated by the National Society for Medical Research and the "Guide for the Care and Use of Laboratory Animals" prepared by the Institute of Laboratory Animal Resource and published by the National Institute of Health (NIH Publication No. 86-23, revised 1985).

Under generalanesthesia withisoflurane and nitrous oxide, a left fifth interspace thoracotomy was done.

Polyethylene catheters were inserted into the thoracic aorta via the left carotid artery for measuring systemic blood pressure (BP) and the left jugular vein for intravenous infusion. Fiberoptic pulmonary artery catheters (Oximetrix; Abbott Critical Care systems, North Chicago, IL) were placed in the pulmonary artery to allow mixed venous oxygen consumption (SvO2) and pulmonary arterial pressure (PAP).

Heart rate (HR) was continuously monitored by electrocardiography.

For measuring aortic blood flow and bypass flow, electromagnetic flowmeters (MF-2100; Nihon-Koden, Tokyo, Japan) were placed in the ascending aorta and LVAD outflow cannulae.

Aortic blood flow was used as an index of native cardiac output, and the VAD assist ratio was calculated as follows.

$$VAD\ assist\ ratio\ (\%) = bypass\ flow\ (l/min.)/(aortic\ blood\ flow\ (l/min.) + bypass\ flow\ (l/min.))$$

The impaired heart was created by ligation of the left anterior descending (LAD) coronary artery distal to its first diagonal branch (Fig. 1, A).

After ligation, all goats underwent cardiogenic shock and developed severe arrhythmias, such as ventricular fibrillation and ventricular tachycardia.

In order to maintain systemic circulation and unload the left ventricle, an LVAD (Toyobo, Osaka, Japan) was installed extracorporeally between the left atrium and the descending aorta.

A 1/2 inch vinyl chloride inflow cannula with multiple side holes was used for blood drainage and a 1/2 inch vinyl chloride outflow cannula with a 12 mm woven graft (Meadox, Oakland, USA) was used for blood return.

This outflow cannula sutured onto the descending aorta and inflow cannula was inserted into left atrium without cardiopulmonary by pass (Fig. 1,B) after systemic heparinization (300 u / kg, intravenous injection).

And an RVAD (Toyobo, Osaka, Japan) was installed extracorporeally between the right atrium and the pulmonary trunk in the same procedure (Fig. 1, B).

The goats were divided into two groups randomly. In the HGF group, hHGF-cDNA plasmid, a total of 2.0 mg hHGF-cDNA in 2.0 ml of plasmid solution, was injected using 30 G needles into the myocardium at ten points of the ischemic area of the left ventricular wall (Fig. 1, A).

There were no changes in the hemodynamic conditions associated with the injection of hHGF-cDNA plasmid, and no obvious adverse effects, such as anaphylactic reaction, in the goats throughout this experiment.

In the control group, an equivalent volume of beta-galactosidase plasmid was injected in the same procedure.

The chest was then closed and allowed to recover from anesthesia.

To detect of hHGF protein in the treated myocardium, two goats from each group were killed three days after the cDNA injection.

In another three goats from each group, systemic circulation was subsequently maintained under BVAD for 4 weeks.

Anticoagulation was performed 2days after surgery.

Warfarin sodium was administrated with the target of the international normalized ratio, which ranged between 2.5-3.5.

No platelet antiaggregation drugs were administrated.

(3) Assessment of cardiac function

We estimated the changes of cardiac function by means of three-dimensional digital sonomicrometry (Sonometrics Corp., Ontario, Canada) [16].

Two ultrasonic crystals were implanted in the endocardium parallel to the short axis at the level of the papillary muscle at the time of operation (Fig. 1, C).

These crystals were placed in the anterior wall and its opposite site to assess myocardial contractility in the distribution of the LAD coronary artery.

The LV dimension at end-diastole (LVDd) and end-systole (LVDs) were determined by simultaneously measured LVP.

The LV percent fractional shortening (%FS) was calculated as follows:

$$\%FS\ (\%) = (LVDd - LVDs)/LVDd \times 100$$

Before and after ligation of the LAD coronary artery, and at 1, 2, 3, and 4 weeks after gene transfection, we measured %FS and cardiac output on the condition that turned off BVAD for short periods.

(4) VAD off test

Four weeks after gene transfection, an attempt was made to wean all goats from BVAD.

After systemic heparinization (300 u / kg, intravenous injection), the BVAD was turned off.

At 5, 15, and 30 minutes after turning off the BVAD, we measured HR, BP, SvO2, PAP, cardiac output, and LVDd.

(5) Histological analysis

Four weeks after plasmid administration and after the VAD off test, all goats were euthanized with an overdose of sodium pentobarbital and the hearts were excised.
The hearts were cut at the short axis into 5 pieces, and LV myocardium specimens were fixed with 10% buffered formalin and embedded in paraffin.
A few serial sections from each specimen were cut into 5-mm-thick slices and stained with hematoxylin and eosin for histological examination and measurement of cardiomyocyte cell diameter or with AZAN-trichrome stain to assess the collagen content.
The proportion of the fibrosis occupying area at the border area neighboring the infarct area was measured on 10 randomly selected fields and the result was expressed as the percent fibrosis.
To label vascular endothelial cells so that the bloodvessels could be counted in the border area neighboring the infarct area, immunohistochemical staining of Von Willebrand Factor-related antigen was performed according to a modified protocol.
We used EPOS-conjugated antibody against Von Willebrand Factor-related antigen coupled with HRP (DAKO EPOS Anti-Human Von Willebrand Factor/HRP, DAKO) as primary antibodies.
The stained vascular endothelial cells were counted as vascular density under a lightmicroscopic at x200 magnification, using at least ten randomly selected fields per section.
The result was expressed as the number of blood vessels/$mm^2$. Computer appraisals of pathology (cell diameter, percent fibrosis and vascular density) were performed by a Macintosh computer using a public domain image program developed at the US National Institute of Health.

(6) Statistical analysis

All data are expressed as [the] mean±standard deviation. Intergroup comparisons were made using ANOVA and the unpaired Student's t-test.
All analyses were performed using the program StatView (version 5.0; Abacus Concepts, Inc., Berkeley, CA).
Values of $p<0.05$ were considered to indicate statistical significance.

Results

(1) In vivo HGF gene transfection

Three days after transfecting hearts with hHGF-cDNA plasmid, we measured the hHGF protein content in the myocardial samples obtained from the cDNA-injected area by an ELISA assay.
The myocardiatransfected with the hHGF-cDNA contained hHGF protein at levels as high as 1.0±0.3 ng / g tissue on the third day after transfection.
In contrast, hHGF was not detected in the myocardia of the control group animals.
The serum hHGF levels were not detected both in the two groups throughout this the experiment.

(2) Animal condition and systemic hemodynamic data

Just after infarction, all goats developed severe low output syndrome and cardiogenic shock.
Native cardiac outputs decreased about 20 or 30 ml/kg/min.
Under the support of BVAD, all animals were maintained in good condition, and the HR and BP on unloaded conditions by VAD support did not differ between the two groups throughout this experiment (Table 1).

Table 1

| | pre | post | 3day | 1w | 2w | 3w | 4w |
|---|---|---|---|---|---|---|---|
| Heart Rate (beats/min.) | | | | | | | |
| HGF group | 98±2 | 59±1 | 145±5 | 146±4 | 131±9 | 121±17 | 118±17 |
| Control group | 86±10 | 62±10 | 140±7 | 147±4 | 134±3 | 113±28 | 90±28 |
| Mean Aortic Pressure (mmHg) | | | | | | | |
| HGF group | 100±10 | 59±8 | 82±3 | 82±5 | 87± 5 | 94± 5 | 91±5 |

(continued)

| | | | | | | | |
|---|---|---|---|---|---|---|---|
| Mean Aortic Pressure (mmHg) | | | | | | | |
| Control group | 96± 6 | 60±10 | 80±7 | 86±9 | 97±10 | 93±10 | 96±3 |

The VAD assist ratio was maintained at about 70% throughout this experiments, and this ratio did not differ markedly between the two groups (Fig. 2, A).

However, at 4 weeks after the gene transfection, the cardiac output in the HGF group was significantly higher than that in the control group (85.0±1.4 ml/kg/min. in the HGF group vs. 64.6±6.0 ml/kg/min. in the control group, $p<0.01$; Fig. 2, B).

(3) Assessment of cardiac function

After infarction, the %FS was markedly decreased compared with the baseline values in both groups, and the degree of deterioration did not differ between two groups.

The %FS were recovered gradually in the two groups after gene tranfection.

However, the improvement of the %FS in the HGF group were significantly larger than the control group.

The %FS in the HGF group 4 weeks after gene transfection was significantly recovered than the control group (37.9±1.7 % in the HGF group vs. 26.4±0.3 % in the control group, $p<0.01$; Fig. 3).

(4) VAD off test

Four weeks after gene transfection, we performed a VAD "off test" (Fig. 4).

HR was steadily increased and BP was steadily decreased in the control group on loaded condition after the BVAD was turned off, but in the HGF group, these parameters remained stable and did not deteriorate.

The SvO2 and the PAP of the control group also deteriorated relative to those of the HGF group.

Cardiac output was significantly increased in the HGF group compared with the control group 30 minutes after weaning from VAD (80.1±6.2ml/kg/min. in the HGF group vs. 61.2±4.3ml/kg/min. in the control group, $p<0.05$; Fig. 4).

And in the control group, LVDd was significantly increased relative to that in the HGF group 30 minutes after weaning from VAD (35.8±1.6 mm in the HGF group vs. 46.9±0.1 mm in the control group, $p<0.05$; Fig. 4).

(5) Histological assessment

Macroscopic findings of expired hearts revealed that LV dilatation was markedly suppressed in the HGF group relative to that in the control group, although necrotic change and scar formation of the anterior wall were recognized in both groups (Fig. 5, A and B).

Azan staining of the myocardium in the neighborhood of the infarcted area revealed that fibrous change was also suppressed in the HGF group compared with that in the control group (Fig. 5, C and D).

The percent fibrosis was significantly reduced in the HGF group compared with the control group (13.9±1.7 % in the HGF group vs. 22.3±1.3 % in the control group, $p<0.01$; Fig. 6, A).

HE staining of the border zone revealed hypertrophic change of cardiomyocytes in the control group, but not in the HGF group (Fig. 5, E and F).

The cell diameter was significantly smaller in the HGF group than in the control group (39.6±0.5 μm in the HGF group vs. 54.4+0.6 μm in the control group, $p<0.01$; Fig. 6, B).

Vascular density was examined in the border zone of the infarct area (Fig. 5, G and H).

Vascular density was significantly higher in the HGF group than in the control group (35.2±2.1 vessels per field in the HGF group vs. 24.5±2.7 vessels per field in the control group, $p<0.05$; Fig. 6, C).

Discussion

(1) left ventricular unloading by VAD alone could not achieve sufficient suppression of cardiac remodeling after myocardial infarction; and

(2) gene transfection with HGF-cDNA plasmid attenuated cardiac remodeling in the impaired heart under mechanical unloading with VAD, and achieved markedly better improvement of cardiac function than that by VAD alone, suggesting its potential use as a "bridge to recovery".

Recently, several studies of regenerative therapy with gene therapy or cell transplantation have reported the effect on protection of cardiomyocytes and improvement of cardiac function in the impaired heart (17-20).

But such therapies require time to take effect, and are not able to control heart failure immediately after treatment.

VAD may not only support systemic circulation but provide an optimal environment for myocardial recovery along with ventricular unloading (2-5, 21-23).

We, therefore, propose a combination therapy consisting of gene therapy and VAD as a new strategy for the treatment of severe heart failure.

VAD provides sufficient time and suitable circumstances for myocardial regeneration, and regenerative therapy promotes myocardial recovery in the impaired heart resulting in the increase of a "bridge to recovery".

HGF is a potent angiogenic factor, and we have started its clinical application at Osaka University Hospital for patients with arteriosclerosis obliterans (24).

Furthermore, HGF is not only an angiogenic factor but also shows various physiological activities, including antifibrotic and cardiotrophic activities (6-9, 25, 26).

Therefore, webelieve thatHGFhas an advantage for promoting myocardial regeneration.

In the chronic phase of myocardial infarction, the progression of cardiac remodeling with reduced cardiac function is responsible for interstitial fibrosis as well as for the apoptosis of the cardiomyocytes.

In particular, fibrosis remote from the infarcted area is considered to be the major cause of ventricular remodeling in ischemic cardiomyopathy.

In this study, neoangiogenesis was induced and fibrosis was suppressed in the peri-infarcted area by HGF gene transfection.

Some of the molecular contributors to fibrosis during cardiac remodeling have been identified (27).

Transforming growth factor-β and angiotensin-II are believed to play an important role in the pathogenesis of fibrosis (28-30).

These molecules are the negative regulators of local HGF production in various cell types (7-9).

In this study, increase of local HGF expression may prevent myocardial fibrosis, possibly by inhibiting the production of such molecules as previously reported (6-10).

Regarding delivery of HGF, we did not use any vector for gene therapy in this study.

Because, we have already reported that direct administration of HGF-cDNA plasmid is enough for local and continuous intramural delivery of HGF to enhance angiogenesis and cardiac function in the infarct myocardium (6-8, 24, 25).

It is speculated that native HGF also plays an important role as a cardio protective factor, but native HGF is insufficient for attenuation of cardiac remodeling in this experiments. Gene transfection of hHGF plasmid is also support the cardioprotective role of native HGF, and thus a lower quantity of continuously expressed protein could be sufficient to induce angiogenesis and support the subsequent recovery of regional cardiac function (13, 14).

Moreover, HGF acts as a paracrine growth factor and its production by administration of HGF-cDNA plasmid in the myocardium continues about for 14 days.

Therefofre, its local synthesis without viral vectors might safe against adverse effects while no detection of in the serum HGF level during gene therapy.

Thus, our results might promise clinical applications.

The present invention is the first report to demonstrate the effectiveness of regenerative therapy in the impaired heart under LVAD, and the protocol of this study can be used as one of the new therapeutic strategies for severe heart failure.

However, several limitations of this study must be considered before developing a clinical application.

First, due to limitations of the experimental protocol, we were not able to clarify the efficacy of this method with respect to scar thinning and expansion of the impairedmyocardium in the chronic phase.

When loss of contractile mass is markedly increased such as in patients with dilated cardiomyopathy, regeneration of cardiomyocytes is insufficient to increase the contractile function even with the HGF gene transfection.

Thus a method of supplementing the contractile mass, such as cellular cardiomyoplasty, may be needed to increase the treatment efficacy.

Second, because of the lack of techniques for measuring goat HGF, the changes and roles of endogenous HGF in this experiment are not clear.

Third, the long-term outcome of the effects of HGF is also unclear.

In the setting of this experiments, after coronary ligation, severe arrythmias such as ventricular tachycardia and fibliration frequently occurred.

So we implanted RVADS in order to maintain the VAD flow and systemic circulation.

In conclusion, we have demonstrated the possible therapeutic value of suppression of cardiac remodeling by hHGF gene transfection in the impaired heart under LVAD.

Our results suggest that, in the setting of acute myocardial infarction causing cardiogenic shock, a combined therapy with HGF gene therapy and LVAD can increase the chance of a "bridge to recovery" in the severely impaired heart under LVAD.

References

1. Hosenpud JD, Bennett LE, Kech BH, Fiol B, Boucek MM, Novick RJ. The registry of the International Society for Heart and Lung Transplantation: Seventeenth Official reports-2000. J Heart Lung Transplant. 2000;19:909-931.
2. Rose EA, Gelijns AC, Moskowitz AJ, Heitjan DF, Stevenson LW, Dembitsky W, et al. Long-term mechanical left ventricular assistance for end-stage heart failure. N Engl J Med. 2001; 345 (20) :1435-43.
3. Frazier OH, Myers T. Left Ventricular Assist System as a Bridge to Myocardial Recovery. Ann Thorac Surg. 1999; 68:734-741
4. LevinHR, OzMC, Chen JM, PackerM, Rose EA, Burkhoff D. Reversal of chronic ventricular dilatation in patients with end-stage cardiomyopathy by prolonged mechanical unloading. Circulation. 1995;91:2717-20.
5. Hetzer R, Muller J, Weng Y, Wallukat G, Spiegelsberger S, Loebe M. Cardiac recovery in dilated cardiomyopathy by unloading with left ventricular assist device. Ann Thorac Surg. 1999;68:742-749.
6. Aoki M, Morishita R, Taniyama Y, Kida I, Moriguchi A, Matsumoto K, et al. Angiogenesis induced by hepatocyte growth factor in non-infarcted myocardium and infarcted myocardium: up-regulation of essential transcription factor for angiogenesis, ets. Gene Ther. 2000;7:417-427.
7. Matsumoto K, Nakamura T. Emerging multipotent aspects of hepatocyte growth factor. J Biochem. 1996;119: 591-600.
8. Taniyama Y, Morishita T, Nakagami H, Moriguchi A, Sakonjo H, Kim S, et al. Potential contribution of a novel antifibrotic factor, hepatocyte growth factor, to prevention of myocardial fibrosis by angiotensin II blockade in cardiomyopathic hamsters. Circulation. 2000;102:246-252.
9. Nakamura T, Mizuno S, Matsumoto K, Sawa Y, Matsuda H, Nakamura T. Myocardial protection from ischemia/ reperfusion injury by endogenous and exogenous HGF. J Clin Invest. 2000;106:1511-1519.
10. Ueda H, Sawa Y, Matsumoto K, Kitagawa-Sakakida S, Kawahira Y, Nakamura T, et al. Gene transfection of hepatocyte growth factor attenuates reperfusion injury in the heart. Ann Thorac Surg. 1999;67:1726-31.
11. Treasure CB, Alexander RW. The dysfunctional endothelium in heart failure. J Am Coll Cardiol. 1993;22: A129-134.
12. Pfeffer MA, Braunwald E. Ventricular remodeling after myocardial infarction. Circulation. 1990;81:1161-1172.
13. Ono k, Matsumori A, Shioi T, Furukawa Y, Sasayama S. Enhanced expression of Hepatocyte Growth Factor / c-Met by myocardial ischemia and reperfusion in a rat model. Circulation. 1997; 95:2552-2558.
14. Ueda H, Nakamura T, Matsumoto K, Sawa Y, Matsuda H, Nakamura T. A potential cardioprotective role of hepatocyte growth factor in myocardial infarction in rats. Cardiovascular Reseach. 2001; 51:41-50.
15. Seki T, Hagiya M, Shimonishi M, Nakamura T, Shimizu S. Organization of human hepatocyte growth factor-encoding gene. Gene. 1991;102:213-219.
16. Smith W, Vesely I. Three dimensional ultrasonic micrometer for use in cardiovascular research. Proceedings of the 13th Conference of the IEEE-EMBS, 1991.
17. Taylor DA, Atkins BZ, Hungspreuges P, Jones TR, Reedy MC, Hutchenson KA, et al. Regenerating functional myocardium: improved performance after skeletal myoblast transplantation. Nat Med 1998;4:929-933.
18. Kocher AA, Schuster MJ, Szabolks MJ, Takuma D, Burkhoff D, Wang J, etal. Neovascularization of ischemic myocardium by human bone-marrow-derived angioblasts prevents cardiomyocyte apoptosis, reduces remodeling and improves cardiac function. Nat Med 2001;7:430-436.
19. Jackson KA, Majka SM, Wang H, Pocius J, Hartley CJ, Goodell MA. Regeneration of ischemic cardiac muscle and vascular endothelium by adult stem cells. J Clin Invest 2001;107:1395-1402.
20. Suzuki K, Murtuza B, Smolenski RT, Sammut IA, Suzuki N, Kaneda Y, et al. Cell transplantation for the treatment of acute myocardial infarction using vascular endothelial growth factor-expressing skeletal myoblasts. Circulation 2001;104[suppl I]:I-207-I-212.
21. Levin HR, Oz MC, Catanese KA, Rose EA, Burkhoff D. Transient normalisation of systolic and diastolic function after support with a left ventricular assist device in a patient with dilated cardiomyopathy. J Heart Lung Transplant. 1996;15:840-842.
22. Frazier OH, Benedect CR, Radovancevic B, Bick RJ, Capek P, Springer WE, et al. Improved left ventricular function after chronic left ventricular unloading. Ann Thorac Surg. 1996;62:675-682.
23. McCarthy PM, Nakatani S, Vargo R, Kottke-Marchant K, Harasaki H, James KB, et al. Structural and left ventricular histologic changes after implantable LVAD insertion. Ann Thorac Surg. 1995;59:609-613.
24. Taniyama Y, Morishita R, Aoki M, Nakagami H, Yamamoto K, Yamazaki K, et al. Therapeutic angiogenesis induced by human hepatocyte growth factor gene in rat and rabbit hindlimb ischemia models: preclinical study for treatment of peripheral arterial disease. Gene Ther. 2001;8:e181-e189.
25. FunatsuT, SawaY, OhtakeS, Takahashi T, MatsumiyaG, Matsuura N, et al. Therapeutic angiogenesis in the ischemic canine heart induced by myocardial injection of naked complementary DNA plasmid encoding hepatocyte growth factor. J Thorac Cardiovasc Surg. 2002; 124:1099-1105.
26. Ono M, Sawa Y, Matsumoto K, Nakamura T, Kaneda Y, Matsuda H. In vivo gene transfection with hepatocyte growth factor via the pulmonary artery induces angiogenesis in the rat lung. Circulation. 2002; 106[suppl I]:I-264-269.
27. Goldstein S, Ali AS, Sabbah H. Ventricular remodeling mechanisms and prevention. Cardiol Clin. 1998;16: 623-631.
28. Kapadia S, Dibbs Z, Kurrelmeyer K, Kalra D, Seta Y, Wang F, et al. The role of cytokines in the failing human heart. Cardiol Clin. 1998;16:645-656.
29. BristowMR, GinsburgR, Umans V, FowlerM, Minobe W, Rasmussen R, et al. β1- and β2- adrenergic-receptor subpopulations in nonfailing and failing human myocardium: coupling of both receptor subtypes and selective β1-receptor down-regulation in heart failure. Circ Res. 1986;59:297-309.
30. Gwathmey JK, Copelas L, MacKinnon R, Schoen FJ, Feldman MD, Grossman W, et al Abnormal intracellular calcium handling in myocardium from patients with end-stage heart failure. Circ Res. 1987;61:70-76.

**Claims**

1. A medicament comprising a gene encoding an angiogenic cytokine for the treatment of acute myocardial infarction (AMI), idiopathic cardiomyopathy (ICM), dilated cardiomyopathy (DCM) or heart failure, to be given in combination with ventricular assist device (VAD).

2. The medicament of claim 1, wherein the angiogenic cytokine is selected from the group consisting of hepatocyte growth factor (HGF), vascular endothelial growth factor (VEGF), fibroblast growth factor (FGF), epidermal growth factor (EGF), nerve growth factor (NGF), transforming growth factor (TGF), platelet derived growth factor (PDGF) and insulin-like growth factor (IGF).

3. The medicament of claim 1 or 2, wherein the angiogenic cytokine is HGF.

4. The medicament in any of claims 1 to 3, wherein the gene is in the form of plasmid.

5. The medicament in any of claims 1 to 4, wherein the angiogenic cytokine is administered directly into the myocardium.

6. The medicament in any of claims 1 to 5, wherein the angiogenic cytokine is injected at plural points in the myocardium.

7. The medicament in any of claims 1 to 6, wherein the myocardium is the ischemic area of the left ventricular wall.

8. The medicament in any of claims 1 to 7, wherein the VAD is left ventricular assist device (LVAD).

9. A method of treating acute myocardial infarction (AMI), idiopathic cardiomyopathy (ICM), dilated cardiomyopathy (DCM) or heart failure, which comprises administering the medicament according to claim 1 to a patient having a ventricular assist device (VAD) in combination.

10. Use of the gene encoding an angiogenic cytokine for producing a medicament for treating acute myocardial infarction (AMI), idiopathic cardiomyopathy (ICM), dilated cardiomyopathy (DCM) or heart failure of a patient having ventricular assist device (VAD) in combination.

Fig. 2

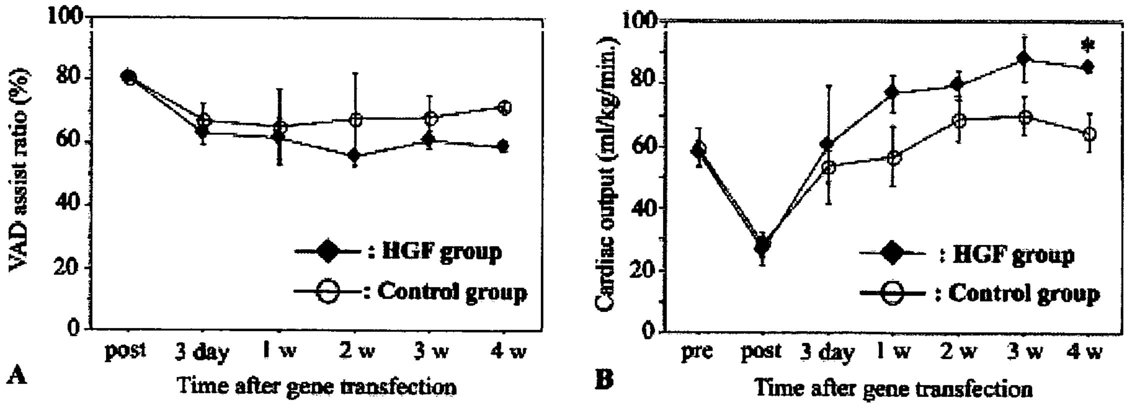
A
VAD assist ratio (%)
100
80
60
40
20
0
post
3 day
1 w
2 w
3 w
4 w
Time after gene transfection
: HGF group
: Control group
B
Cardiac output (ml/kg/min.)
pre
post
*


Fig. 3

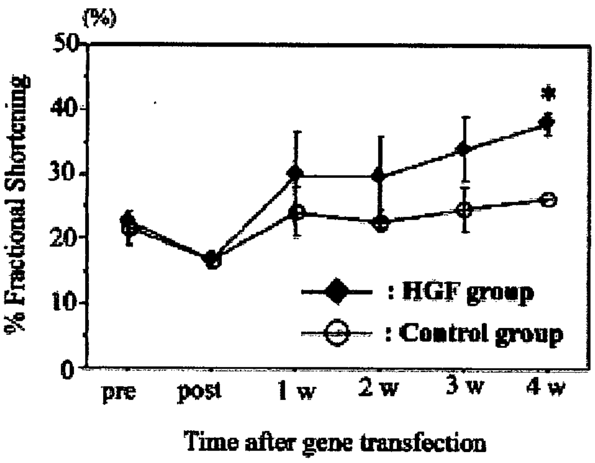
(%)
% Fractional Shortening
50
40
30
20
10
0
pre
post
1 w
2 w
3 w
4 w
*
: HGF group
: Control group
Time after gene transfection

Fig. 4

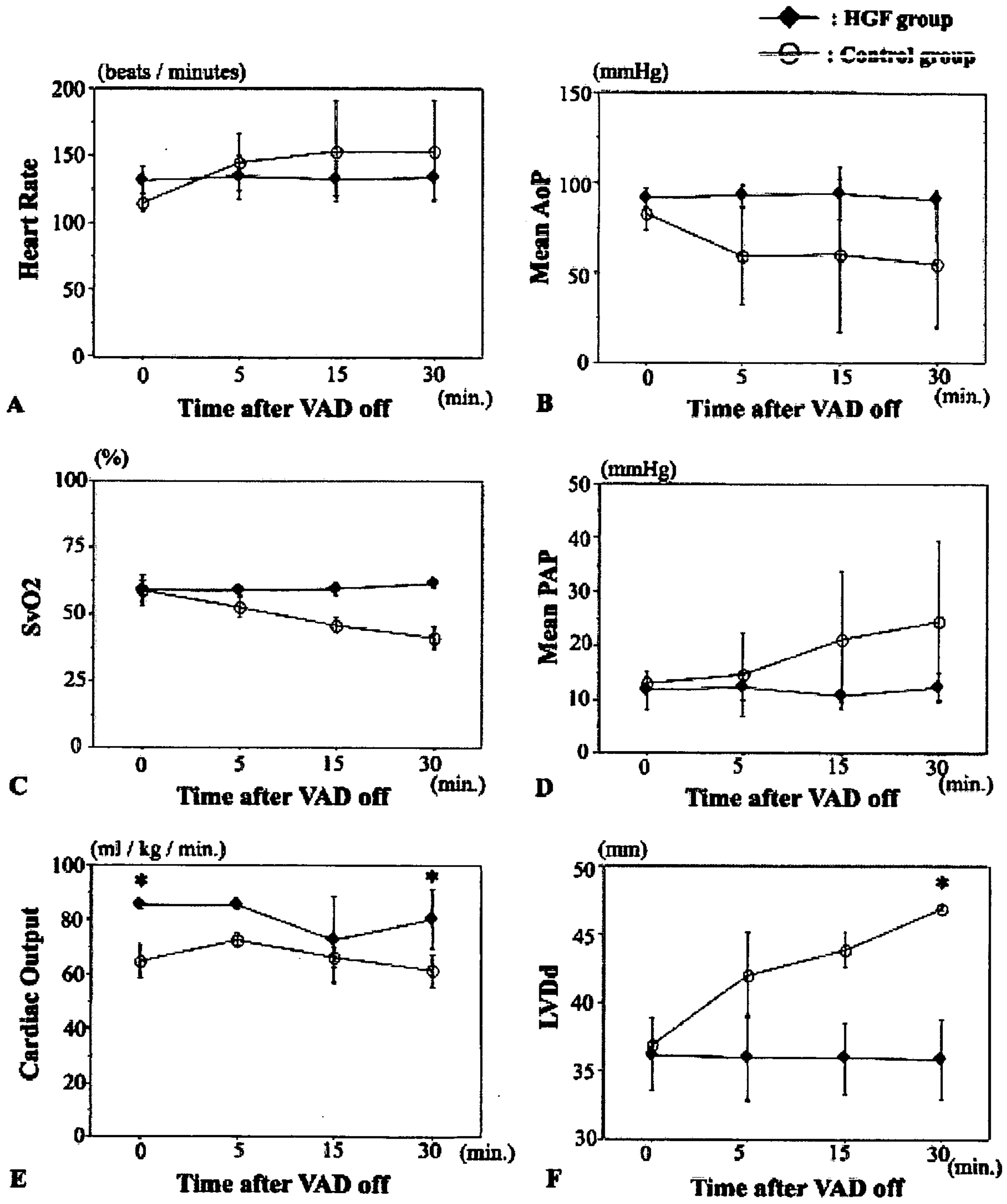
◆ : HGF group
○ : Control group
(beats / minutes)
Heart Rate
200
150
100
50
0
0
5
15
30
(min.)
Time after VAD off
A
(mmHg)
Mean AoP
150
100
50
0
0
5
15
30
(min.)
Time after VAD off
B
(%)
SvO2
100
75
50
25
0
0
5
15
30
(min.)
Time after VAD off
C
(mmHg)
Mean PAP
50
40
30
20
10
0
0
5
15
30
(min.)
Time after VAD off
D
(ml / kg / min.)
Cardiac Output
100
80
60
40
20
0
*
*
0
5
15
30
(min.)
Time after VAD off
E
(mm)
LVDd
50
45
40
35
30
*
0
5
15
30
(min.)
Time after VAD off
F

Fig. 6

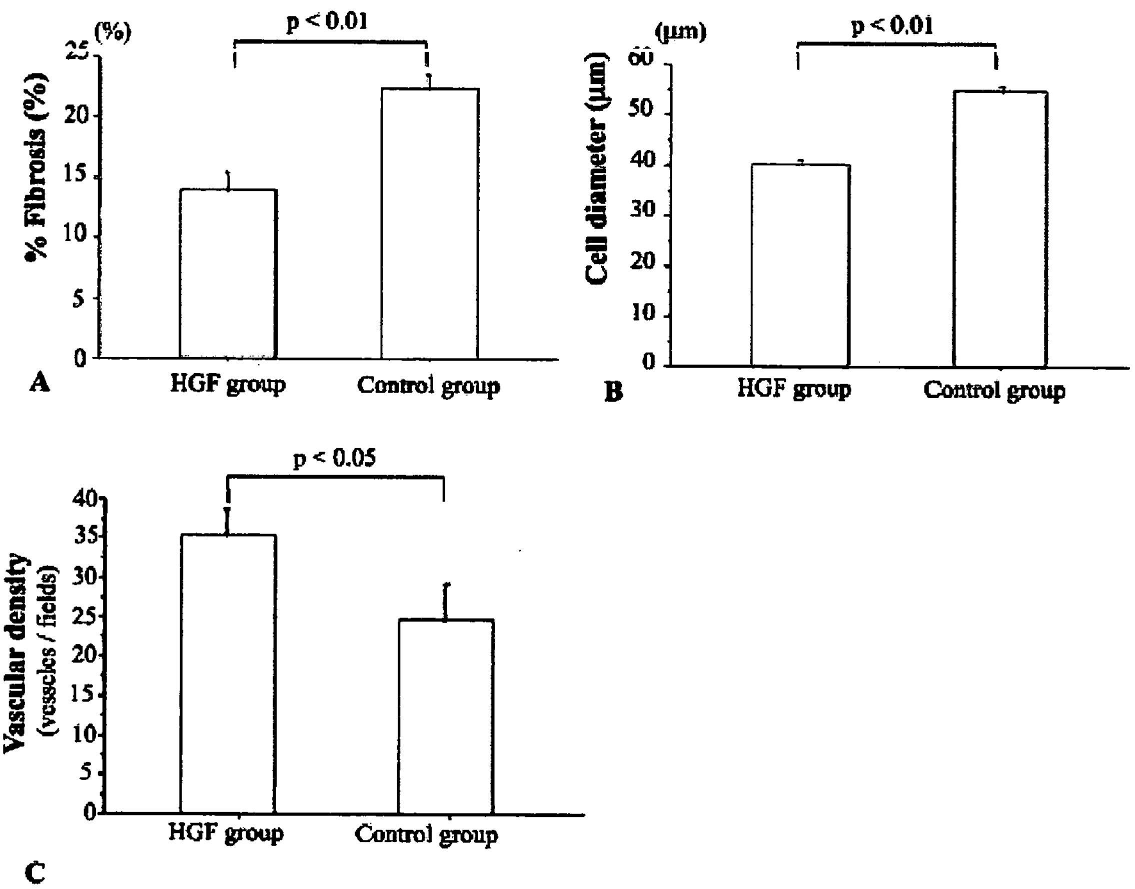
A
(%)
% Fibrosis (%)
p < 0.01
0
5
10
15
20
25
HGF group
Control group
B
(μm)
Cell diameter (μm)
p < 0.01
0
10
20
30
40
50
60
HGF group
Control group
C
Vascular density
(vessicles / fields)
p < 0.05
0
5
10
15
20
25
30
35
40
HGF group
Control group

**INTERNATIONAL SEARCH REPORT**

International application No.
PCT/JP2005/020163

A. CLASSIFICATION OF SUBJECT MATTER
*A61K48/00*(2006.01), *A61K38/00*(2006.01), *A61P9/00*(2006.01), *A61P9/04*(2006.01), *A61P9/10*(2006.01)

According to International Patent Classification (IPC) or to both national classification and IPC

B. FIELDS SEARCHED

Minimum documentation searched (classification system followed by classification symbols)
*A61K48/00*(2006.01), *A61K38/00*(2006.01),

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
REGISTRY/CA/MEDLINE/EMBASE/BIOSIS(STN), JSTPlus/JMEDPlus(JOIS)

C. DOCUMENTS CONSIDERED TO BE RELEVANT

| Category* | Citation of document, with indication, where appropriate, of the relevant passages | Relevant to claim No. |
|---|---|---|
| X | Hiromi KOIKE et al., "6. Kekkan Shinsei to Saisei Iryo", Annual Review Junkanki 2002, 2002, pages 38 to 44 | 1-8,10 |
| X | Hiromi KOIKE et al., "Kekkan Kino no Saisei", Kagaku Kogyo, 2002, The December issue, pages 925 to 929 | 1-8,10 |
| X | Yoshiki SAWA, "Zoki Fuzen Chiryo no Atarashii Hoko Jusho Shinfuzen ni Taisuru Shinkin Saisei Chiryo", Gendai Iryo, 2001, Vol.33, pages 2928 to 2932 | 1-8,10 |
| X | Yoshiki SAWA, "Junkanki Geka ni Okeru Idenshi Chiryo", Igaku no Ayumi, 2000, Vol.192, No.3, pages 229 to 232 | 1-8,10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 December, 2005 (12.12.05) | 20 December, 2005 (20.12.05) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No. PCT/JP2005/020163

C (Continuation). DOCUMENTS CONSIDERED TO BE RELEVANT

| Category* | Citation of document, with indication, where appropriate, of the relevant passages | Relevant to claim No. |
|---|---|---|
| X | Yoshiki SAWA, "2. Shinkin ni Okeru Saibo Chiryo", Experimental Medicine, 2003, Vol.21, No.8, pages 1116 to 1121 | 1-8,10 |
| X | Yoshiki SAWA, "Kyoketsu Shinkin ni Taisuru Idenshi Chiryo to Rinsho Kogaku", Clinical Engineering, 2003, Vol.14, No.2, pages 167 to 171 | 1-8,10 |
| X | Hikaru MATSUDA, "Jusho Shinfuzen Chiryo ni Okeru Hojo Jinko Shinzo no Yakuwari", The Japanese Journal of Artificial Organs, 2003, Vol.32, No.1, pages 7 to 12 | 1-8,10 |
| Y | Yoshinori MITAMURA, "Jinko Shinzo (Kiso)", The Japanese Journal of Artificial Organs, 2003, Vol.31, No.3, pages 75 to 78 | 1-8,10 |

Form PCT/ISA/210 (continuation of second sheet) (April 2005)

**INTERNATIONAL SEARCH REPORT**

International application No.
PCT/JP2005/020163

**Box No. II Observations where certain claims were found unsearchable (Continuation of item 2 of first sheet)**

This international search report has not been established in respect of certain claims under Article 17(2)(a) for the following reasons:

1. [X] Claims Nos.: 9
because they relate to subject matter not required to be searched by this Authority, namely:
Claim 9 pertains to methods for treatment of the human body by therapy and thus relates to a subject matter which this International Searching Authority is not required, under the provisions of the PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

**INTERNATIONAL SEARCH REPORT**

International application No.
PCT/JP2005/020163

<Subject of prior art search>

The language "angiogenesis cytokine" recited as an active ingredient in claims 1, 2, 4-8 and 10 covers a vast variety of substances. However, it appears that only HGF is disclosed within the meaning of PCT Article 5, so that support within the meaning of PCT Article 6 is lacking. Consequently, in this international search report, prior art reference search has been restricted to active ingredients particularly described in the description.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 01026694 A

### Non-patent literature cited in the description


- **HOSENPUD JD ; BENNETT LE ; KECH BH ; FIOL B ; BOUCEK MM ; NOVICK RJ.** The registry of the International Society for Heart and Lung Transplantation: Seventeenth Official reports-2000. *J Heart Lung Transplant.,* 2000, vol. 19, 909-931
- **ROSE EA ; GELIJNS AC ; MOSKOWITZ AJ ; HEITJAN DF ; STEVENSON LW ; DEMBITSKY W et al.** Long-term mechanical left ventricular assistance for end-stage heart failure. *N Engl J Med.,* 2001, vol. 345 (20), 1435-43
- **FRAZIER OH ; MYERS T.** Left Ventricular Assist System as a Bridge to Myocardial Recovery. *Ann Thorac Surg.,* 1999, vol. 68, 734-741
- **LEVINHR ; OZMC ; CHEN JM ; PACKERM ; ROSE EA ; BURKHOFF D.** Reversal of chronic ventricular dilatation in patients with end-stage cardiomyopathy by prolonged mechanical unloading. *Circulation,* 1995, vol. 91, 2717-20
- **HETZER R ; MULLER J ; WENG Y ; WALLUKAT G ; SPIEGELSBERGER S ; LOEBE M.** Cardiac recovery in dilated cardiomyopathy by unloading with left ventricular assist device. *Ann Thorac Surg.,* 1999, vol. 68, 742-749
- **AOKI M ; MORISHITA R ; TANIYAMA Y ; KIDA I ; MORIGUCHI A ; MATSUMOTO K et al.** Angiogenesis induced by hepatocyte growth factor in non-infarcted myocardium and infarcted myocardium: up-regulation of essential transcription factor for angiogenesis, ets. *Gene Ther.,* 2000, vol. 7, 417-427
- **MATSUMOTO K ; NAKAMURA T.** Emerging multipotent aspects of hepatocyte growth factor. *J Biochem.,* 1996, vol. 119, 591-600
- **TANIYAMA Y ; MORISHITA T ; NAKAGAMI H ; MORIGUCHI A ; SAKONJO H ; KIM S et al.** Potential contribution of a novel antifibrotic factor, hepatocyte growth factor, to prevention of myocardial fibrosis by angiotensin II blockade in cardiomyopathic hamsters. *Circulation,* 2000, vol. 102, 246-252
- **NAKAMURA T ; MIZUNO S ; MATSUMOTO K ; SAWA Y ; MATSUDA H ; NAKAMURA T.** Myocardial protection from ischemia/reperfusion injury by endogenous and exogenous HGF. *J Clin Invest.,* 2000, vol. 106, 1511-1519
- **UEDA H ; SAWA Y ; MATSUMOTO K ; KITAGAWA-SAKAKIDA S ; KAWAHIRA Y ; NAKAMURA T et al.** Gene transfection of hepatocyte growth factor attenuates reperfusion injury in the heart. *Ann Thorac Surg.,* 1999, vol. 67, 1726-31
- **TREASURE CB ; ALEXANDER RW.** The dysfunctional endothelium in heart failure. *J Am Coll Cardiol.,* 1993, vol. 22, A129-134
- **PFEFFER MA ; BRAUNWALD E.** Ventricular remodeling after myocardial infarction. *Circulation,* 1990, vol. 81, 1161-1172
- **ONO K ; MATSUMORI A ; SHIOI T ; FURUKAWA Y ; SASAYAMA S.** Enhanced expression of Hepatocyte Growth Factor / c-Met by myocardial ischemia and reperfusion in a rat model. *Circulation,* 1997, vol. 95, 2552-2558
- **UEDA H ; NAKAMURA T ; MATSUMOTO K ; SAWA Y ; MATSUDA H ; NAKAMURA T.** A potential cardioprotective role of hepatocyte growth factor in myocardial infarction in rats. *Cardiovascular Reseach.,* 2001, vol. 51, 41-50
- **SEKI T ; HAGIYA M ; SHIMONISHI M ; NAKAMURA T ; SHIMIZU S.** Organization of human hepatocyte growth factor-encoding gene. *Gene,* 1991, vol. 102, 213-219
- **SMITH W ; VESELY I.** Three dimensional ultrasonic micrometer for use in cardiovascular research. *Proceedings of the 13th Conference of the IEEE-EMBS,* 1991
- **TAYLOR DA ; ATKINS BZ ; HUNGSPREUGES P ; JONES TR ; REEDY MC ; HUTCHENSON KA et al.** Regenerating functional myocardium: improved performance after skeletal myoblast transplantation. *Nat Med,* 1998, vol. 4, 929-933

- **KOCHER AA ; SCHUSTER MJ ; SZABOLKS MJ ; TAKUMA D ; BURKHOFF D ; WANG J et al.** Neovascularization of ischemic myocardium by human bone-marrow-derived angioblasts prevents cardiomyocyte apoptosis, reduces remodeling and improves cardiac function. *Nat Med,* 2001, vol. 7, 430-436
- **JACKSON KA ; MAJKA SM ; WANG H ; POCIUS J ; HARTLEY CJ ; GOODELL MA.** Regeneration of ischemic cardiac muscle and vascular endothelium by adult stem cells. *J Clin Invest,* 2001, vol. 107, 1395-1402
- **SUZUKI K ; MURTUZA B ; SMOLENSKI RT ; SAMMUT IA ; SUZUKI N ; KANEDA Y et al.** Cell transplantation for the treatment of acute myocardial infarction using vascular endothelial growth factor-expressing skeletal myoblasts. *Circulation,* 2001, vol. 104 (I), I-207-I-212
- **LEVIN HR ; OZ MC ; CATANESE KA ; ROSE EA ; BURKHOFF D.** Transient normalisation of systolic and diastolic function after support with a left ventricular assist device in a patient with dilated cardiomyopathy. *J Heart Lung Transplant.,* 1996, vol. 15, 840-842
- **FRAZIER OH ; BENEDECT CR ; RADOVANCEVIC B ; BICK RJ ; CAPEK P ; SPRINGER WE et al.** Improved left ventricular function after chronic left ventricular unloading. *Ann Thorac Surg.,* 1996, vol. 62, 675-682
- **MCCARTHY PM ; NAKATANI S ; VARGO R ; KOTTKE-MARCHANT K ; HARASAKI H ; JAMES KB et al.** Structural and left ventricular histologic changes after implantable LVAD insertion. *Ann Thorac Surg.,* 1995, vol. 59, 609-613
- **TANIYAMA Y ; MORISHITA R ; AOKI M ; NAKAGAMI H ; YAMAMOTO K ; YAMAZAKI K et al.** Therapeutic angiogenesis induced by human hepatocyte growth factor gene in rat and rabbit hindlimb ischemia models: preclinical study for treatment of peripheral arterial disease. *Gene Ther.,* 2001, vol. 8, e181-e189
- **FUNATSUT ; SAWAY ; OHTAKES ; TAKAHASHI T ; MATSUMIYAG ; MATSUURA N et al.** Therapeutic angiogenesis in the ischemic canine heart induced by myocardial injection of naked complementary DNA plasmid encoding hepatocyte growth factor. *J Thorac Cardiovasc Surg.,* 2002, vol. 124, 1099-1105
- **ONO M ; SAWA Y ; MATSUMOTO K ; NAKAMURA T ; KANEDA Y ; MATSUDA H.** In vivo gene transfection with hepatocyte growth factor via the pulmonary artery induces angiogenesis in the rat lung. *Circulation,* 2002, vol. 106 (I), I-264-269
- **GOLDSTEIN S ; ALI AS ; SABBAH H.** Ventricular remodeling mechanisms and prevention. *Cardiol Clin.,* 1998, vol. 16, 623-631
- **KAPADIA S ; DIBBS Z ; KURRELMEYER K ; KALRA D ; SETA Y ; WANG F et al.** The role of cytokines in the failing human heart. *Cardiol Clin.,* 1998, vol. 16, 645-656
- **BRISTOWMR ; GINSBURGR ; UMANS V ; FOWLERM ; MINOBE W ; RASMUSSEN R et al.** β1- and β2- adrenergic-receptor subpopulations in nonfailing and failing human myocardium: coupling of both receptor subtypes and selective β1-receptor down-regulation in heart failure. *Circ Res.,* 1986, vol. 59, 297-309
- **GWATHMEY JK ; COPELAS L ; MACKINNON R ; SCHOEN FJ ; FELDMAN MD ; GROSSMAN W et al.** Abnormal intracellular calcium handling in myocardium from patients with end-stage heart failure. *Circ Res.,* 1987, vol. 61, 70-76